(19) Europäisches Patentamt
European Patent Office
Office européen des brevets

(11) **EP 1 813 628 A1**

(12) **EUROPEAN PATENT APPLICATION**
published in accordance with Art. 158(3) EPC

(43) Date of publication:
**01.08.2007 Bulletin 2007/31**

(21) Application number: **05806059.1**

(22) Date of filing: **11.11.2005**

(51) Int Cl.:
*C08F 20/06* (2006.01)     *A61F 5/44* (2006.01)
*A61F 13/49* (2006.01)     *A61F 13/53* (2006.01)
*A61L 15/60* (2006.01)     *B01J 20/26* (2006.01)
*C08F 20/56* (2006.01)

(86) International application number:
**PCT/JP2005/020690**

(87) International publication number:
**WO 2006/054487 (26.05.2006 Gazette 2006/21)**

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **17.11.2004 JP 2004333275**

(71) Applicant: **SUMITOMO SEIKA CHEMICALS CO.,
LTD.
Kako-gun, Hyogo 675-0145 (JP)**

(72) Inventors:
• **HANDA, Masayoshi Sumitomo Seika Chem. Co
Ltd.
Hyogo 672-8076 (JP)**
• **NAWATA, Yasuhiro Sumitomo Seika Chem. Co
Ltd.
Hyogo 672-8076 (JP)**

(74) Representative: **Vossius & Partner
Siebertstrasse 4
81675 München (DE)**

(54) **WATER ABSORBING RESIN PARTICLE AND, MAKING USE OF THE SAME, ABSORBER MATERIAL AND ABSORBENT ARTICLE**

(57)     Water-absorbent resin particles, **characterized in that** the water-absorbent resin particles have a water-retention capacity of physiological saline (X) of from 30 to 70 g/g, that the water-retention capacity of physiological saline (X) and a water absorption index under pressure (Y) satisfy the relationship of the formula: [Water absorption index under pressure (Y)] $\geq$ 150 - 2 [Water-retention capacity of physiological saline (X)], and that the water-absorbent resin particles have a liquid-suction rate from pulp of 20 g/g/5 minutes or more; and an absorbent material and an absorbent article, each in which the water-absorbent resin particles are used. The absorbent material in which the water-absorbent resin particles are used can be suitably used, for example, in absorbent articles such as disposable diapers and sanitary napkins.

EP 1 813 628 A1

**Description**

TECHNICAL FIELD

The present invention relates to water-absorbent resin particles, and an absorbent material and an absorbent article each in which water-absorbent resin particles are used. More specifically, the present invention relates to water-absorbent resin particles which are excellent in water-retention capacity (an absorption capability), water absorption capability under pressure, and a liquid-suction rate, and an absorbent material and an absorbent article each in which the water-absorbent resin particles are used.

BACKGROUND ART

Absorbent articles such as disposable diapers and sanitary napkins comprise an absorbent material comprising a hydrophilic fiber and a water-absorbent resin. The absorbent material is an important component for absorbing and retaining a liquid such as urine or blood.

Amounts and a ratio of a hydrophilic fiber and a water-absorbent resin in an absorbent material vary depending upon the purpose of use and the product design of an absorbent article. For example, in disposable diapers, the weight of absorbent materials varies depending upon their sizes such as S, M, and L, and the contents of the water-absorbent resin in the absorbent material also are in a variety, ranging from those containing smaller volumes to those containing larger volumes.

In view of the background as described above, it has been desired for the absorbent material that stable properties can be obtained even while the contents of the water-absorbent resin in the absorbent material undergo various changes, and that excellent properties of the absorbent material are exhibited even in any use embodiments, for example, disposable diapers, for urination in various bodily positions such as a sitting position and a standing position.

The main roles of the hydrophilic fiber when the absorbent material is allowed to absorb a liquid such as urine or blood are to rapidly absorb a liquid at an initial stage and to widely diffuse the liquid. On the other hand, the main roles of the water-absorbent resin are to suck the liquid rapidly absorbed by the hydrophilic fiber, to retain the sucked liquid in a larger amount, and to absorb the liquid under a load. It has been desired that the water-absorbent resin has an appropriate level of an absorption capacity, a water absorptive rate, a liquid-suction force, water absorption capability under pressure, gel strength, or the like in order that the absorbent material exhibits excellent properties. On the other hand, since the hydrophilic fiber is poor in properties other than a liquid-suction rate, the above-mentioned properties of the water-absorbent resin are essentially important factors for determining advantageousness and disadvantageousness of the absorbent materials.

In order to solve the problems of the above-mentioned absorbent material, some techniques for the preparation of the water-absorbent resin have been proposed, for the purpose of improving the properties of the water-absorbent resin. For example, as a process for preparing a water-absorbent resin having a high absorption capacity and high water absorption capability under pressure, a process comprising carrying out reversed phase suspension polymerization in the copresence of a hypophosphorous acid compound (see, for example, Patent Publication 1); a process comprising carrying out polymerization in the copresence of a hypophosphorous acid and/or a salt thereof and an urea compound (see, for example, Patent Publication 2); a process comprising carrying out reversed phase suspension polymerization in the copresence of a β-1,3-glucan to give a water-absorbent resin, and further adding a crosslinking agent to the resulting water-absorbent resin, to carry out a crosslinking reaction (see, for example, Patent Publication 3); a process comprising carrying out reversed phase suspension polymerization using a specified amount of a persulfuric acid salt as a polymerization initiator (see, for example, Patent Publication 4); a process comprising carrying out reversed phase suspension polymerization in the presence of a crosslinking agent and a water-soluble chain transfer agent (see, for example, Patent Publication 5); or the like has been known. However, the properties of the absorbent materials in which the above water-absorbent resins are used are not satisfactory at all.

Patent Publication 1: Japanese Patent Laid-Open No. Hei 11-255837

Patent Publication 2: Japanese Patent Laid-Open No. Hei 6-145253

Patent Publication 3: Japanese Patent Laid-Open No. Hei 8-120013

Patent Publication 4: Japanese Patent Laid-Open No. Hei 6-287233

Patent Publication 5: Japanese Patent Laid-Open No. Hei 2-255804

DISCLOSURE OF INVENTION

PROBLEMS TO BE SOLVED BY THE INVENTION

A first object of the present invention is to provide water-absorbent resin particles which are excellent in water-retention capacity, water absorption capability under pressure, and a liquid-suction rate. A second object of the present invention is to provide an absorbent material and an absorbent article which stably exhibit excellent properties even while the contents of the water-absorbent resin particles undergo various changes.

MEANS TO SOLVE THE PROBLEMS

The present invention relates to:

(1) water-absorbent resin particles, characterized in that the water-absorbent resin particles have a water-retention capacity of physiological saline (X) of from 30 to 70 g/g, that the water-retention capacity of physiological saline (X) and a water absorption index under pressure (Y) satisfy the relationship of the formula:

$$[\text{Water absorption index under pressure (Y)}] \geq 150 - 2[\text{Water-retention capacity of physiological saline (X)}],$$

and that the water-absorbent resin particles have a liquid-suction rate from pulp of 20 g/g/5 minutes or more;
(2) an absorbent material comprising the water-absorbent resin particles as defined above, and a hydrophilic fiber; and
(3) an absorbent article comprising the absorbent material as defined above interposed between a liquid-permeable sheet and a liquid-impermeable sheet.

EFFECTS OF THE INVENTION

The water-absorbent resin particles of the present invention are excellent in water-retention capacity, water absorption capacity under pressure, and a liquid-suction rate. The absorbent material and the absorbent article of the present invention stably exhibit excellent properties even while the contents of the water-absorbent resin particles undergo various changes.

BRIEF DESCRIPTION OF THE DRAWINGS

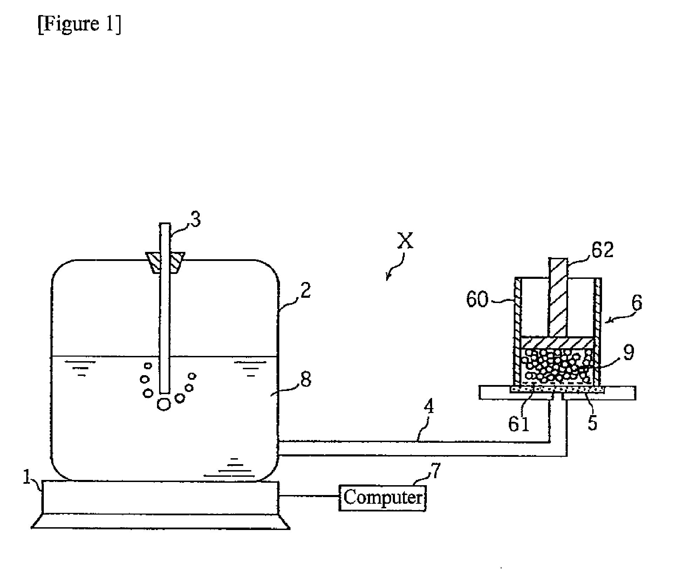

[Figure 1] A schematic view showing an outline of the constitution of an apparatus for determining the water absorption capacity under pressure.

EXPLANATION OF NUMERICAL SYMBOLS

X measuring apparatus
1 balance
2 bottle
3 air aspiration tube
4 lead tube
5 glass filter
6 measuring section
60 cylinder (of measuring section)
61 nylon mesh (of measuring section)
62 weight (of measuring section)
7 computer
8 physiological saline
9 water-absorbent resin particles

BEST MODE FOR CARRYING OUT THE INVENTION

The water-absorbent resin particles of the present invention have a water-retention capacity of physiological saline (X) of from 30 to 70 g/g, preferably from 35 to 65 g/g, and more preferably from 40 to 60 g/g. In a case where the water-absorbent resin particles have a water-retention capacity of physiological saline of less than 30 g/g, an absorption capacity of an absorbent material and an absorbent article is lowered when they are produced. Therefore, a large amount of liquid cannot be entirely absorbed therein, so that liquid leakage, re-wet or the like is generated, whereby the feel of comfortable fit of the absorbent article is likely to be worsened. In addition, in a case where the water-absorbent resin particles have a water-retention capacity of physiological saline exceeding 70 g/g, the deterioration of gel is likely to be generated with the passage of time after the liquid absorption. Therefore, a liquid containing a mucous component is eluted, whereby the feel of comfortable fit of the absorbent article is also likely to be worsened.

The water-retention capacity of physiological saline (X) of the water-absorbent resin particles is a value determined in accordance with the determination method described in "(2) Water-Retention Capacity of Physiological Saline of Water-Absorbent Resin Particles" given later.

The water absorption index under pressure (Y) of the water-absorbent resin particles of the present invention means a total sum of water absorption capacities of physiological saline of the water-absorbent resin particles under the pressures of 2.07 kPa, 4.14 kPa, and 6.21 kPa. Here, the pressures are set at 2.07 kPa, 4.14 kPa, and 6.21 kPa based on the reason that the water-absorbent resin particles have an ability of absorbing a liquid in a larger amount even in the state that the absorbent material is exposed to various levels of loads by having a fitted individual of the absorbent article take a bodily position such as a sitting position or a standing position.

The water absorption index under pressure (Y) of the water-absorbent resin particles is a value determined in accordance with the determination method described in "(3) Water Absorption Capacity Under Pressure and Water Absorption Index Under Pressure of Water-Absorbent Resin Particles" given later.

The water-retention capacity of physiological saline (X) and the water absorption index under pressure (Y) of the water-absorbent resin particles of the present invention satisfy the relationship of the formula:

$$[\text{Water absorption index under pressure (Y)}] \geq 150 - 2[\text{Water-retention capacity of physiological saline (X)}], \text{ and}$$

preferably satisfying the relationship of the formula:

$$[\text{Water absorption index under pressure (Y)}] \geq 160 - 2[\text{Water-retention capacity of physiological saline (X)}].$$

In a case where the water-retention capacity of physiological saline (X) and the water absorption index under pressure (Y) do not satisfy the above-mentioned formula, liquid leakage and re-wet are more likely to be generated, wherein the liquid leakage and re-wet are caused by poor absorbency of the water-absorbent resin particles when the absorbent material is exposed to various levels of loads, especially when a load is increased by having a fitted individual take a posture such as a sitting position.

Here, the reason why the above-mentioned formula is set is based on the matter that the water-absorbent resin particles satisfying the above formula have excellent absorption property (a smaller amount of leakage) and a small amount of re-wet, even while the contents of the water-absorbent resin particles in the absorbent material undergo various changes.

The water-absorbent resin particles of the present invention have a liquid-suction rate from pulp of preferably 20 g/g/5 minutes or more, and more preferably 25 g/g/5 minutes or more. In a case where the water-absorbent resin particles have a liquid-suction rate from pulp of less than 20 g/g/5 minutes, in an absorbent material comprising pulp and water-absorbent resin particles, and an absorbent article in which the absorbent material is used, the water-absorbent resin particles cannot rapidly absorb a liquid absorbed in and diffused into the pulp, so that the liquid leakage or the amount of re-wet from pulp increases, thereby making it more likely to lead to the worsening of the feel of comfortable fit of an absorbent article.

Here, the reason why the liquid-suction rate from pulp has been set is based on the matter that when a liquid is provided to the absorbent material, it is preferable that the water-absorbent resin particles are a substance which absorbs a liquid retained in the pulp after the liquid diffusion at a substantially fast rate of absorption.

The liquid-suction rate from pulp of the water-absorbent resin particles is a value determined in accordance with the determination method described in "(4) Liquid-Suction Rate and Equilibrium Liquid-Suction Amount" given later.

The water-absorbent resin particles of the present invention have the equilibrium liquid-suction amount from pulp of preferably 30 g/g or more, and more preferably 35 g/g or more. In a case where the water-absorbent resin particles have the equilibrium liquid-suction amount from pulp of less than 30 g/g, the capacity of the liquid that the water-absorbent resin particles are capable of substantially absorbing in an absorbent material comprising pulp and the water-absorbent resin particles, and an absorbent article in which the absorbent material is used, becomes smaller, so that a liquid remains in the pulp, whereby the amount of re-wet is increased when a load after the absorption is increased, thereby making it likely to lead to the worsening of the feel of comfortable fit of an absorbent article.

Here, the reason why the equilibrium liquid-suction amount from pulp has been set is based on the matter that when a liquid is provided to the absorbent material, it is preferable that the equilibrium is achieved after the water-absorbent resin particles absorb a liquid retained by pulp after the liquid diffusion in a substantially larger amount.

The equilibrium liquid-suction amount from pulp of the water-absorbent resin particles is a value determined in accordance with the determination method of "Equilibrium Liquid-Suction Amount" described in "(4) Liquid-Suction Rate and Equilibrium Liquid-Suction Amount" given later.

The water-retention capacity of physiological saline (X) and the water absorption index under pressure (Y) satisfying the above-mentioned formula, and the liquid-suction rate from pulp and the equilibrium liquid-suction amount from pulp can be established at a given value by, for example, adding a phosphorus-containing compound thereto only in at least one polymerization step in the second and subsequent steps and properly controlling the water content of a water-containing gel upon addition of a crosslinking agent when a post-crosslinking is further carried out, in a preparation process for obtaining the water-absorbent resin particles by a reversed phase suspension polymerization which is carried out in two or more multi-steps.

The process for preparing water-absorbent resin particles of the present invention is not particularly limited. The process includes, for example, a process for preparing water-absorbent resin particles comprising the steps of carrying out a reversed phase suspension polymerization in two or more multi-steps in the preparation of the water-absorbent resin particles obtained by subjecting a water-soluble ethylenically unsaturated monomer to reversed phase suspension polymerization, the process comprising adding a phosphorus-containing compound thereto only in at least one step in the second and subsequent steps to carry out the polymerization reaction, and the like.

In the above-mentioned process, a first-step reversed phase suspension polymerization reaction in a water-in-oil system is firstly carried out by the method including the steps of mixing an aqueous solution of a water-soluble ethylenically unsaturated monomer, a surfactant and/or a polymeric protective colloid, a water-soluble radical polymerization initiator, and a hydrocarbon-based solvent, and heating the mixture with stirring.

The water-soluble ethylenically unsaturated monomer includes, for example, (meth)acrylic acid ["(meth)acryl-" means "acryl-" and/or "methacryl-;" hereinafter referred to the same], 2-(meth)acrylamide-2-methylpropanesulfonic acid or an alkali metal salt thereof; nonionic unsaturated monomers such as (meth)acrylamide, N,N-dimethylacrylamide, 2-hydroxyethyl (meth)acrylate, and N-methylol (meth)acrylamide; amino group-containing unsaturated monomers such as diethylaminoethyl (meth)acrylate and diethylaminopropyl (meth)acrylate, or a quaternary salt thereof; and the like. These can be used alone or in admixture of two or more kinds. The alkali metal in the alkali metal salts includes lithium, sodium, potassium, and the like.

Among the water-soluble ethylenically unsaturated monomers, preferred ones include (meth)acrylic acid and an alkali metal salt thereof, acrylamide, methacrylamide and N,N-dimethylacrylamide, from the viewpoint of being industrially easily available. Even more preferred ones include (meth)acrylic acid and an alkali metal salt thereof, from the viewpoint of economical advantage.

The water-soluble ethylenically unsaturated monomer can be usually used in the form of an aqueous solution. It is preferable that the concentration of the water-soluble ethylenically unsaturated monomers in the aqueous solution of the water-soluble ethylenically unsaturated monomers is from 15% by mass to a saturated concentration.

In the aqueous solution of the water-soluble ethylenically unsaturated monomer, when the water-soluble ethylenic monomer to be used has an acid group, the acid group may be neutralized with an alkali metal, or the like. It is preferable that the degree of neutralization by the alkali metal is from 10 to 100% by mol of the acid group of the water-soluble ethylenically unsaturated monomer before the neutralization, from the viewpoint of increasing osmotic pressure and increasing water absorption rate of the resulting water-absorbent resin particles, and not causing any disadvantages in safety or the like due to the presence of an excess alkali metal. The alkali metal includes lithium, sodium, potassium, and the like. Among them, sodium and potassium are preferable, and sodium is more preferable.

The surfactant includes, for example, nonionic surfactants such as sorbitan fatty acid esters, polyglycerol fatty acid esters, sucrose fatty acid esters, sorbitol fatty acid esters, and polyoxyethylene alkylphenyl ethers; anionic surfactants such as fatty acid salts, alkylbenzenesulfonic acid salts, alkyl methyl tauric acid salts, polyoxyethylene alkylphenyl ether sulfate esters, and polyoxyethylene alkyl ether sulfonic acid salts; and the like. Among them, sorbitan fatty acid esters, polyglycerol fatty acid esters, and sucrose fatty acid esters are preferable.

The polymeric protective colloid includes, for example, ethyl cellulose, ethyl hydroxyethyl cellulose, polyethylene oxide, maleic anhydride-modified polyethylene, maleic anhydride-modified polybutadiene, maleic anhydride-modified EPDM (ethylene-propylene-diene terpolymer), and the like.

The surfactant and/or the polymeric protective colloid is contained in an amount of preferably from 0.1 to 5 parts by mass, and more preferably from 0.2 to 3 parts by mass, based on 100 parts by mass of the aqueous solution of the water-soluble ethylenically unsaturated monomer.

The water-soluble radical polymerization initiator includes, for example, persulfates such as potassium persulfate, ammonium persulfate, and sodium persulfate; organic peroxides such as tert-butyl hydroperoxide, cumene hydroperoxide, and benzoyl peroxide; hydrogen peroxide; azo compounds such as 2,2'-azobis(2-amidinopropane)dihydrochloride; and the like. Among them, potassium persulfate, ammonium persulfate, sodium persulfate, benzoyl peroxide, and 2,2'-azobis(2-amidinopropane)dihydrochloride are preferable, from the viewpoint of being easily available and excellent in storage stability. The water-soluble radical polymerization initiator can be used as a redox-system polymerization initiator together with a sulfite or the like.

It is preferable that the water-soluble radical polymerization initiator is contained in an amount of usually from 0.00005 to 0.01 mol per 1 mol of the water-soluble ethylenically unsaturated monomer, from the viewpoint of shortening the time period for the polymerization reaction and preventing abrupt polymerization reaction.

The hydrocarbon-based solvent includes, for example, aliphatic hydrocarbons such as n-hexane and n-heptane; alicyclic hydrocarbons such as cyclopentane, methylcyclopentane, cyclohexane, and methylcyclohexane; aromatic hydrocarbons such as benzene, toluene, and xylene; and the like. Among them, n-hexane, n-heptane, and cyclohexane are preferable, from the viewpoint of being industrially easily available, stable in quality and inexpensive.

Usually, the hydrocarbon-based solvent is contained in an amount of preferably from 50 to 600 parts by mass, and more preferably from 80 to 550 parts by mass, based on 100 parts by mass of the water-soluble ethylenically unsaturated monomer, from the viewpoint of removing heat of polymerization and being likely to easily control the polymerization temperature.

In the present invention, it is preferable that the polymerization reaction is carried out in the presence of an internal crosslinking agent. The internal crosslinking agent includes, for example, unsaturated polyesters obtained by reacting polyols such as diols or triols such as (poly)ethylene glycol ["(poly)" means cases where the prefix "poly" is included and where the prefix is not included; hereinafter referred to the same], (poly)propylene glycol, 1,4-butanediol, trimethylolpropane, or (poly)glycerol with an unsaturated acid such as (meth)acrylic acid, maleic acid or fumaric acid; bisacrylamides such as N,N'-methylenebisacrylamide; di- or tri(meth)acrylate esters obtained by reacting a polyepoxide with (meth)acrylic acid; carbamyl esters of di(meth)acrylic acid obtained by reacting a polyisocyanate such as tolylene diisocyanate or hexamethylene diisocyanate with hydroxyethyl (meth)acrylate; compounds each having two or more polymerizable unsaturated groups, such as allylated starch, allylated cellulose, diallyl phthalate, N,N',N''-triallyl isocyanurate, and divinylbenzene; diglycidyl ether compounds such as (poly)ethylene glycol diglycidyl ether, (poly)propylene glycol diglycidyl ether, and (poly)glycerol diglycidyl ether; epihalohydrin compounds such as epichlorohydrin, epibromohydrin, and α-methylepichlorohydrin; compounds each having two or more reactive functional groups, such as isocyanate compounds such as 2,4-tolylene diisocyanate and hexamethylene diisocyanate; oxetane compounds such as 3-methyl-3-oxetanemethanol, 3-ethyl-3-oxetanemethanol, 3-butyl-3-oxetanemethanol, 3-methyl-3-oxetancethanol, 3-ethyl-3-oxetaneethanol, and 3-butyl-3-oxetaneethanol; and the like. These can be used alone, or in admixture of two or more kinds.

It is preferable that the internal crosslinking agent is contained in an amount of preferably from 0.000001 to 0.001 mol per 1 mol of the water-soluble ethylenically unsaturated monomer in order to suppress the water solubility of the resulting polymer by an appropriate crosslinking of the polymer, thereby showing a satisfactory water absorbency.

The reaction temperature of the polymerization reaction differs depending upon the radical polymerization initiator used. The reaction temperature is preferably from 20° to 110°C and more preferably from 40° to 90°C, from the viewpoint of rapidly progressing the polymerization and shortening the polymerization time, thereby increasing productivity, and easily removing heat of polymerization, thereby smoothly carrying out the reaction. The reaction time is usually from 0.5 to 4 hours.

Thus, the first-step reversed phase suspension polymerization is carried out.

Next, the reaction mixture obtained by the first-step reversed phase suspension polymerization is subjected to second- and subsequent-steps reversed phase suspension polymerization. In the present invention, the reversed phase suspension polymerization is carried out in multi-steps of two or more steps, and it is preferable that the number of steps is two or three steps from the viewpoint of increasing productivity.

In the present invention, it is preferable that the reversed phase suspension polymerization is carried out in the presence of a phosphorus-containing compound only in at least one step of the second and subsequent steps. The process for carrying out a reversed phase suspension polymerization in the second and subsequent steps in the presence of a phosphorus-containing compound is not particularly limited. One example of the process for carrying out a reversed phase suspension polymerization in the second and subsequent steps includes a process comprising adding an aqueous solution of a water-soluble ethylenically unsaturated monomer to the reaction mixture obtained in the first-step polymerization reaction with mixing, and carrying out a reversed phase suspension polymerization in the second and subsequent steps in the same manner as in the first step.

During the reaction, the phosphorus-containing compound may be added to the aqueous solution of a water-soluble ethylenically unsaturated monomer which is usable when carrying out the reversed phase suspension polymerization in the second and subsequent steps, or may be added to the reaction mixture obtained by the reversed phase suspension polymerization in the first and subsequent steps after cooling.

The phosphorus-containing compound is not particularly limited. The phosphorus-containing compound includes, for example, phosphorous acid compounds such as phosphorous acid, normal salts of phosphorous acid such as disodium phosphite, dipotassium phosphite, and diammonium phosphite, and acidic salts of phosphorous acid such as sodium hydrogenphosphite, potassium hydrogenphosphite, and ammonium hydrogenphosphite; phosphoric acid compounds such as phosphoric acid, normal salts of phosphoric acid such as sodium phosphate, potassium phosphate, and ammonium phosphate, and acidic salts of phosphoric acid such as sodium dihydrogenphosphate, potassium dihydrogenphosphate, ammonium dihydrogenphosphate, disodium hydrogenphosphate, dipotassium hydrogenphosphate, and diammonium hydrogenphosphate; hypophosphorous acid compounds such as hypophosphorous acid and salts of hypophosphorous acid such as sodium hypophosphite, potassium hypophosphite, and ammonium hypophosphite; pyrophosphoric acid, tripolyphosphoric acid, polyphosphoric acid, and salts thereof; trimethyl phosphate, nitrilotrimethylene triphosphonic acid, and the like. Each of these phosphorus-containing compounds may be used alone or in a mixture of two or more kinds. In addition, as the phosphorus-containing compound, a hydrate of these compounds may be used. Among the phosphorus-containing compounds, the phosphorous acid compounds, the phosphoric acid compounds, and the hypophosphorous acid compounds are preferable, and disodium phosphite, sodium dihydrogenphosphate, and sodium hypophosphite are more preferable, from the viewpoint that an effect exhibited by its addition is high.

It is desired that the phosphorus-containing compound is contained in an amount of from 0.00001 to 0.05 mol, preferably from 0.00005 to 0.02 mol, and even more preferably from 0.0001 to 0.01 mol, per 1 mol of the water-soluble ethylenically unsaturated monomer usable in the polymerization reaction in the step of carrying out the polymerization reaction by adding the phosphorus-containing compound to the reaction mixture in the reversed phase suspension polymerization in the second and subsequent steps. When the phosphorus-containing compound is contained in an amount of less than 0.00001 mol per 1 mol of the water-soluble ethylenically unsaturated monomer, the effect of adding the phosphorus-containing compound is less likely to be sufficiently exhibited. When the phosphorus-containing compound is contained in an amount exceeding 0.05 mol, the amount of mucous component (soluble content) after the liquid absorption is likely to be large.

In the present invention, it is preferable that a post-crosslinking treatment is carried out by reacting the resulting water-absorbent resin particles with a post-crosslinking agent containing two or more functional groups having reactivity with carboxyl groups.

The post-crosslinking agent may be any one that is reactive with a carboxyl group of the water-absorbent resin particles. Representative examples of the post-crosslinking agent include polyols such as (poly)ethylene glycol, (poly) propylene glycol, 1,4-butanediol, trimethylolpropane, and (poly)glycerol; diglycidyl ether compounds such as (poly)ethylene glycol diglycidyl ether, (poly)propylene glycol diglycidyl ether and (poly)glycerol diglycidyl ether; epihalohydrin compounds such as epichlorohydrin, epibromohydrin and $\alpha$-methylepichlorohydrin; compounds each having two or more reactive functional groups, such as isocyanate compounds such as 2,4-tolylene diisocyanate and hexamethylene diisocyanate; and the like. These post-crosslinking agents can be used alone or in a mixture of two or more kinds.

The amount of the post-crosslinking agent cannot be unconditionally determined because the amount differs depending upon the kinds of the post-crosslinking agents. Usually, it is desired that the post-crosslinking agent is contained in an amount of from 0.00001 to 0.01 mol, preferably from 0.00005 to 0.005 mol, and more preferably from 0.0001 to 0.002 mol, per 1 mol of the total amount of the water-soluble ethylenically unsaturated monomer used for the polymerization. When the post-crosslinking agent used is contained in an amount of less than 0.00001 mol, the water-absorbent resin particles are less likely to achieve a satisfactorily high crosslinking density. When the post-crosslinking agent used is contained in an amount exceeding 0.01 mol, the amount of the post-crosslinking agent becomes excessive, so that an unreacted post-crosslinking agent is likely to remain. The timing of addition of the post-crosslinking agent is not particularly limited, and the post-crosslinking agent may be added after the polymerization reaction of the monomer.

It is preferable that the water-absorbent resin particles and the post-crosslinking agent are mixed together in the presence of water. The amount of water upon the mixing the water-absorbent resin particles and the post-crosslinking agent differs depending upon the kinds, particle sizes, and water content of the water-absorbent resin particles. Usually, it is desired that water is contained in an amount of from 5 to 300 parts by mass, preferably from 10 to 100 parts by mass, and more preferably from 10 to 50 parts by mass, based on 100 parts by mass of the total amount of the water-soluble ethylenically unsaturated monomer used for polymerization. When the water is contained in an amount of less than 5 parts by mass, based on 100 parts by mass of the total amount of the water-soluble ethylenically unsaturated monomer used for polymerization, the crosslinking reaction is less likely to progress, and when the water is contained in an amount exceeding 300 parts by mass, the drying time is extended, thereby making it likely to be economically disadvantageous. The amount of water in the present invention means the total amount of water contained upon the polymerization reaction and water used as occasion demands upon the addition of the post-crosslinking agent.

After the past-crosslinking treatment, water and the hydrocarbon-based solvent are distilled off from the water-absorbent resin particles by distillation, whereby a dry product of the water-absorbent resin particles can be obtained.

The absorbent material of the present invention comprises water-absorbent resin particles and a hydrophilic fiber. The constitution of the absorbent material includes, for example, a mix structure in which water-absorbent resin particles and a hydrophilic fiber are homogeneously blended; a sandwich structure in which water-absorbent resin particles are interposed between layers of hydrophilic fibers; a structure in which water-absorbent resin particles and a hydrophilic fiber are wrapped with tissue paper; and the like, and the present invention is not limited only to those exemplified ones. The absorbent material of the present invention may contain a synthetic fiber as a reinforcing material.

The water-absorbent resin particles are contained in an amount of preferably from 5 to 80% by mass, more preferably from 10 to 70% by mass, and even more preferably from 15 to 60% by mass, of the absorbent material. When the water-absorbent resin particles are contained in an amount of less than 5% by mass, an absorption capacity of the absorbent material is lowered, thereby making it likely to lead to increases in the liquid leakage and the amount of re-wet. In addition, when the water-absorbent resin particles are contained in an amount exceeding 80% by mass, the absorbent material is likely to be costly, or the feel of the absorbent material is likely to be toughened.

The hydrophilic fiber includes, for example, cellulose fibers such as cotton pulp obtained from wood, mechanical pulp, chemical pulp and semi-chemical pulp; artificial cellulose fibers such as rayon and acetate; and the like, and the present invention is not limited only to those exemplified ones. The hydrophilic fiber may contain a fiber made of a synthetic resin such as a polyamide, a polyester, or a polyolefin.

The absorbent article of the present invention has a structure in which the above absorbent material is interposed between a liquid-permeable sheet (top sheet) which is capable of passing the aqueous liquid therethrough and a liquid-impermeable sheet (back sheet) which is incapable of passing the aqueous liquid therethrough. The liquid-permeable sheet is placed on a side contacting a body, and the liquid-impermeable sheet is placed on a side not contacting the body.

The liquid-permeable sheet includes, for example, nonwoven fabrics made of a polyethylene, a polypropylene, a polyester, a polyamide or the like; porous synthetic resin sheets; and the like. The liquid-impermeable sheet includes, for example, films made of a synthetic resin such as a polyethylene, a polypropylene or a polyvinyl chloride; films made of composite materials comprising a synthetic resin mentioned above and a nonwoven fabric; and the like, and the present invention is not limited only to those exemplified ones.

The size of the liquid-permeable sheet and the liquid-impermeable sheet cannot be unconditionally determined since the size differs depending upon the applications of the absorbent articles. Therefore, it is preferable that the size is appropriately adjusted in accordance with its purposes.

EXAMPLES

The present invention will be further specifically described hereinbelow by means of Examples, without intending to limit the scope of the present invention thereto.

Example 1

The amount 340 g of n-heptane and 0.92 g of a sucrose fatty acid ester (manufactured by Mitsubishi-Kagaku Foods Corporation under the trade name of S-370) having an HLB of 3.0 were added to a 1000 mL-five-necked cylindrical round bottomed flask equipped with a stirrer, a reflux condenser, a dropping funnel, a thermometer and a nitrogen gas inlet tube. While the mixture was dispersed in the flask, the temperature of the dispersion was raised to 70°C to dissolve the mixture. Thereafter, the resulting solution was cooled to 55°C.

Separately from the above, 92 g of an 80% by mass aqueous solution of acrylic acid (1.02 mol) was added to a 500 mL-Erlenmeyer flask. The amount 102.2 g of a 30% by mass aqueous sodium hydroxide (0.77 mol) was added dropwise to this flask with cooling from external, to neutralize 75% by mol of acrylic acid. Further, 50.2 g of water, 0.11 g (0.00041 mol) of a water-soluble radical polymerization initiator potassium persulfate, and 8.3 mg (0.000047 mol) of a crosslinking agent ethylene glycol diglycidyl ether were added thereto, to give an aqueous monomer solution for a first-step polymerization.

The entire amount of this aqueous monomer solution for a first-step polymerization was added to the above-mentioned five-necked cylindrical round bottomed flask with stirring, and the mixture was dispersed. The internal of the system was sufficiently replaced with nitrogen gas, and the polymerization reaction was carried out for 1 hour while keeping its bath temperature at 70°C. Thereafter, the resulting reaction mixture in the form of a slurry was cooled to room temperature.

Separately from the above, 119.1 g of an 80% by mass aqueous solution of acrylic acid (1.32 mol) was added to a separate 500 mL-Erlenmeyer flask. The amount 132.2 g of a 30% by mass aqueous sodium hydroxide (0.99 mol) was added dropwise thereto with cooling, to neutralize 75% by mol of acrylic acid. Further, 27.4 g of water, 0.14 g (0.00052 mol) of potassium persulfate and 0.54 g (0.0025 mol) of disodium phosphite pentahydrate were added thereto, to give an aqueous monomer solution for a second-step polymerization. The aqueous monomer solution was cooled in an ice water bath.

The entire amount of this aqueous monomer solution for a second-step polymerization was added to the reaction mixture obtained above. Thereafter, the internal of the system was again sufficiently replaced with nitrogen gas, and the second-step polymerization reaction was carried out for 2 hours while keeping its bath temperature at 70°C. After the polymerization reaction, the reaction mixture was heated in an oil bath at 120°C, and the heated mixture was subjected to azeotropic distillation to distill off only 261 g of water to external of the system, to give a gelated product. The amount of remaining water in the gelated product at this point was 56 g.

The amount 7.81 g of a 2% by mass aqueous solution of ethylene glycol diglycidyl ether was added to the resulting gelated product with mixing. Water and n-heptane were further distilled off from the mixture, and the residue was dried, to give 223.1 g of water-absorbent resin particles having a mass-average particle size of 380 $\mu$m.

Example 2

The amount 340 g of n-heptane and 0.92 g of a sucrose fatty acid ester (manufactured by Mitsubishi-Kagaku Foods Corporation under the trade name of S-370) having an HLB of 3.0 were added to a 1000 mL-five-necked cylindrical round bottomed flask equipped with a stirrer, a reflux condenser, a dropping funnel, a thermometer and a nitrogen gas inlet tube. The mixture was dispersed in the flask, and the temperature of the dispersion was raised to 70°C to dissolve the mixture. Thereafter, the resulting solution was cooled to 55°C.

Separately from the above, 92 g of an 80% by mass aqueous solution of acrylic acid (1.02 mol) was added to a 500 mL-Erlenmeyer flask. The amount 102.2 g of a 30% by mass aqueous sodium hydroxide (0.77 mol) was added dropwise to this flask with cooling from external, to neutralize 75% by mol of acrylic acid. Further, 50.2 g of water, 0.11 g (0.00041 mol) of a water-soluble radical polymerization initiator potassium persulfate, and 8.3 mg (0.000047 mol) of a crosslinking agent ethylene glycol diglycidyl ether were added thereto, to give an aqueous monomer solution for a first-step polymerization.

The entire amount of this aqueous monomer solution for a first-step polymerization was added to the above-mentioned five-necked cylindrical round bottomed flask with stirring, and the mixture was dispersed. The internal of the system was sufficiently replaced with nitrogen gas, and the polymerization reaction was carried out for 1 hour while keeping its bath temperature at 70°C. Thereafter, the resulting reaction mixture in the form of a slurry was cooled to room temperature.

Separately from the above, 119.1 g of an 80% by mass aqueous solution of acrylic acid (1.32 mol) was added to a separate 500 mL-Erlenmeyer flask. The amount 132.2 g of a 30% by mass aqueous sodium hydroxide (0.99 mol) was added dropwise thereto with cooling, to neutralize 75 % by mol of acrylic acid. Further, 27.4 g of water, 0.14 g (0.00052 mol) of potassium persulfate and 0.018 g (0.00017 mol) of sodium hypophosphite monohydrate were added thereto, to give an aqueous monomer solution for a second-step polymerization. The aqueous monomer solution was cooled in an ice water bath.

The entire amount of this aqueous monomer solution for a second-step polymerization was added to the reaction mixture obtained above. Thereafter, the internal of the system was again sufficiently replaced with nitrogen gas, and the second-step polymerization reaction was carried out for 2 hours while keeping its bath temperature at 70°C. After the polymerization reaction, the reaction mixture was heated in an oil bath at 120°C, and the heated mixture was subjected to azeotropic distillation to distill off only 258 g of water to external of the system, to give a gelated product. The amount of remaining water in the gelated product at this point was 59 g.

The amount 7.81 g of a 2% by mass aqueous solution of ethylene glycol diglycidyl ether was added to the resulting gelated product with mixing. Water and n-heptane were further distilled off from the mixture, and the residue was dried, to give 223.6 g of water-absorbent resin particles having a mass-average particle size of 382 $\mu$m.

Example 3

The same procedures as in Example 1 were carried out except that the amount dehydrated by azeotropic distillation before the addition of the 2% by mass aqueous solution of ethylene glycol diglycidyl ether in Example 1 was changed to 245 g, to give 224.2 g of water-absorbent resin particles having a mass-average particle size of 382 $\mu$m.

Example 4

The same procedures as in Example 1 were carried out except that the amount of disodium phosphite pentahydrate in Example 1 was changed to 0.71 g (0.0033 mol), to give 224.7 g of water-absorbent resin particles having a mass-average particle size of 375 $\mu$m.

Comparative Example 1

The same procedures as in Example 1 were carried out except that disodium phosphite pentahydrate in Example 1 was not added thereto, to give 220.9 g of water-absorbent resin particles having a mass-average particle size of 380 $\mu$m.

Comparative Example 2

The same procedures as in Example 2 were carried out except that sodium hypophosphite monohydrate in Example 2 was not added thereto, to give 222.3 g of water-absorbent resin particles having a mass-average particle size of 384 $\mu$m.

Comparative Example 3

The same procedures as in Example 4 were carried out except that disodium phosphite pentahydrate in Example 4 was not added thereto, and further that the amount dehydrated by azeotropic distillation before the addition of the 2% by mass aqueous solution of ethylene glycol glycidyl ether in Example 4 was changed to 271 g, to give 220.5 g of water-absorbent resin particles having a mass-average particle size of 374 $\mu$m.

The physical properties of the water-absorbent resin particles obtained in each Example and each Comparative Example were evaluated in accordance with the following methods. The results are shown in Table 1.

(1) Mass-Average Particle Size

One-hundred grams of water-absorbent resin particles were weighed and placed on an uppermost sieve of 8 standard sieves complying with JIS-Z 8801(1982) (openings of sieves: 850 $\mu$m, 500 $\mu$m, 355 $\mu$m, 300 $\mu$m, 250 $\mu$m, 180 $\mu$m, 106 $\mu$m and the bottom of the container being stacked in order from the top), and shaken with a rotating and tapping shaker machine for 10 minutes to sieve the water-absorbent resin particles, and thereafter the water-absorbent resin particles remaining on each sieve was weighed. In accordance with the results, the particle size of which cumulative mass is 50% was calculated in accordance with the following formula:

$$[\text{Mass-Average Particle Size}] = [(50 - a)/(d - a)] \times (c - b) + b$$

wherein a is the cumulative value (g) obtained when the mass is sequentially accumulated from those having coarser particle sizes in the distribution, to obtain a cumulative value of which cumulative mass is less than 50% by mass and most closely approximating 50% by mass; b is an opening ($\mu$m) of the sieve at which the cumulative value is obtained; d is the cumulative value (g) obtained when the mass is sequentially accumulated from those having coarser particle sizes in the distribution to obtain a cumulative value of which cumulative mass is 50% by mass or more and most closely approximating 50% by mass; and c is an opening ($\mu$m) of the sieve at which the cumulative value is obtained.

(2) Water-Retention Capacity of Physiological Saline of Water-Absorbent Resin Particles

The amount 2.0 g of water-absorbent resin particles were weighed in a cotton bag (Cottonbroad No. 60, width 100 mm x length 200 mm), and placed in a 500 mL-beaker. Physiological saline was poured into the cotton bag in an amount of 500 g at one time, and the saline was dispersed so as not to generate an unswollen lump of the water-absorbent resin particles. The upper part of the cotton bag was tied up with a rubber band, and the cotton bag was allowed to stand for 1 hour, to sufficiently swell the water-absorbent resin particles. The cotton bag was dehydrated for 1 minute with a dehydrator (manufactured by Kokusan Enshinki Co., Ltd., product number: H-122) set to have a centrifugal force of 167G, and the mass Wa (g) of the cotton bag containing swelled gels after the dehydration was determined. The same procedures were carried out without adding water-absorbent resin particles, and the empty mass Wb (g) of the cotton bag upon wetting was determined. The water-retention capacity was calculated from the following formula:

$$\text{Water-Retention Capacity g/g}$$
$$= [Wa - Wb]\ (g)/\ \text{Mass of Water-Absorbent Resin Particles (g)}.$$

(3) Water Absorption Capacity Under Pressure and Water

Absorption Index Under Pressure of Water-Absorbent Resin Particles In order to obtain a water absorption index under pressure, water absorption capacities are determined under three kinds of pressures (2.07 kPa, 4.14 kPa, and 6.21 kPa). The determination method of the water absorption capacity under the pressure of 2.07 kPa is exemplified hereinbelow.

The water absorption capacity under pressure was determined using a measuring apparatus X of which outline of the constitution is shown in Figure 1.

The measuring apparatus X shown in Figure 1 comprises a balance 1, a bottle 2 placed on the balance 1, an air aspiration tube 3, a lead tube 4, a glass filter 5, and a measuring section 6 placed on the glass filter 5.

The balance 1 is connected to a computer 7 so that change in mass can be recorded in the units of seconds or minutes. The bottle 2 holds physiological saline in the internal thereof, and the air aspiration tube 3 is inserted in an opening on its top, and the lead tube 4 is attached to the body section. The lower end of the air aspiration tube 3 is soaked in the physiological saline 8. The glass filter 5 has a diameter of 25 mm. As the glass filter 5, Glass Filter No. 1 of SOGO LABORATORY GLASS WORKS CO., LTD. (pore size: 100 to 160 μm) is used. The bottle 2 and the glass filter 5 are communicated with each other via the lead tube 4. In addition, the glass filter 5 is fixed to a position slightly higher than the lower end of the air aspiration tube 3 for the purpose that a liquid amount corresponding to an amount taken away from the filter is provided from the bottle 2 via the lead tube 4.

The measuring section 6 has a cylinder 60, a nylon mesh 61 adhered to the bottom of the cylinder 60, and a weight 62 having the mass of 59.8 g and an outer diameter of 19 mm. The cylinder 60 has an inner diameter of 20 mm. The nylon mesh 61 is formed to have a size of 200 mesh screen (size of opening: 75 μm), and a given amount of water-absorbent resin particles 9 is evenly spread over the nylon mesh 61. The weight 62 is placed on the water-absorbent resin particles 9, so that a 2.07 kPa load can be applied to the water-absorbent resin particles 9.

In the measuring apparatus X having the constitution as described above, first, the bottle 2 was charged with physiological saline in a given amount, and the air aspiration tube 3 was placed in the bottle 2 to get ready for the determination. Next, 0.10 g of the water-absorbent resin particles 9 were evenly spread over the nylon mesh 61 in the cylinder 60, and the weight 62 was placed on the water-absorbent resin particles 9. The measuring section 6 was placed on the glass filter 5 so that its central section is in alignment with the central section of the glass filter 5.

On the other hand, the computer 7 connected to the electronic balance 1 was booted before the determination, and the mass reduction of the physiological saline in the bottle 2, i.e., the mass of the physiological saline absorbed by the water-absorbent resin particles 9, Wc (g), was continuously recorded on the computer 7 from the time point where the water absorption was started, in units of minutes and preferably in units of seconds, on the basis of the value obtained from the balance 1. The water absorption capacity of the water-absorbent resin particles 9 under pressure after 60 minutes from the beginning of the water absorption was obtained by dividing the mass reduction Wc (g) after 60 minutes by the mass of the water-absorbent resin particles 9 (0.10 g).

The determination was carried out in the same manner as in the above-mentioned determination method except that the weight 62 was changed from 59.8 g to 119.6 g in the above-mentioned determination method, to obtain the water absorption capacity of physiological saline under the pressure of 4.14 kPa.

Further, the determination was carried out in the same manner as in the above-mentioned determination method except that the weight 62 was changed from 59.8 g to 179.4 g, to obtain the water absorption capacity of physiological saline under the pressure of 6.21 kPa.

The sum total of the water absorption capacities of physiological saline under the above-mentioned three kinds of the pressures (2.07 kPa, 4.14 kPa, and 6.21 kPa) was defined as a water absorption index under pressure of the water-absorbent resin particles.

(4) Liquid-Suction Rate and Equilibrium Liquid-Suction Amount

A nylon mesh sheet having a sieve opening of 75 μm (diameter: 8 cm) was spread over a glass petri dish having an inner diameter of 9 cm, and a plexiglass cylinder A having an inner diameter of 7.1 cm was placed on top of the sheet. Four grams of pulverized pulp was evenly packed in the internal of the cylinder A, and a plexiglass cylinder B having an inner diameter of 6.0 cm and an outer diameter of 7.0 cm, the cylinder B being attached with a metal mesh having a sieve opening of 180 μm at bottom was inserted from the top of the pulp packing. The cylinder B having the mass of 80 g is movable upwardly and downwardly in the internal of the cylinder A without any resistance.

Sixty grams of physiological saline was poured into the glass petri dish, and allowed to be completely absorbed from the bottom of the pulp via the mesh sheet. Thereafter, a 120 g weight having a diameter of 7 cm was placed on the cylinder B, and allowed to stand for 30 minutes. The weight was removed, and 0.5 g of water-absorbent resin particles classified to particle sizes from 500 to 250 μm were quickly added from the top of the cylinder B so that the resin particles were dispersed on the metal mesh as even as possible. At the same time, the stopwatch was clicked to start, to measure the liquid-suction time. From the mass of a cylinder B containing the water-absorbent resin particles which were allowed to absorb the liquid while varying the liquid-suction time to 5 minutes, 60 minutes, 120 minutes, 180 minutes, and 240 minutes, respectively, the mass of the blank to which the water-absorbent resin particles were not added was subtracted, and the capacity of liquid-suction ($D_{time}$) at each liquid-suction time was measured.

Here, the mass of the blank refers to the mass of the cylinder B after being subjected to a liquid-suction test without the addition of the water-absorbent resin particles. The liquid-suction rate from pulp and the equilibrium liquid-suction amount from pulp are calculated on the bases of the following formulas.

Liquid-suction rate from pulp (g/g/5 minutes) = $D_5$/0.5 Equilibrium liquid-suction amount from pulp (g/g) = $D_{eq}$/0.5

In the formulas, $D_5$ is a value corresponding to the capacity of liquid-suction at 5 minutes; and $D_{eq}$ is the capacity of liquid-suction at a time point where the capacity of liquid-suction reached equilibrium, which was selected between a value corresponding to that at 60 minutes and a value corresponding to that at 240 minutes. A time point where a percentage of increase in the capacity of liquid-suction 60 minutes after a liquid-suction time (t) ($D_{t+60}$), the liquid-suction time (t) being chosen from a time point 60 minutes or later from the start of the liquid-suction, as compared to the capacity of liquid-suction at the liquid-suction time (t) ($D_t$), is less than 5% (i.e., $D_{t+60} < D_t \times 1.05$), and where the liquid-suction time (t) is the shortest is defined as the liquid-suction time at equilibrium (eq), and the liquid-suction amount at that point is defined as ($D_{eq}$). Here, in the case where the above-mentioned requirements are not met, a value corresponding to that at 240 minutes was adopted ($D_{eq} = D_{240}$).

[Table 1]

| Ex. No. and Comp. Ex. No. | Water-Retention Capacity of Physiological Saline | Capacity of Water Absorption Under Pressure (g/g) | | | Water Absorption Index Under Pressure | Liquid-Suction Rate (g/g/5 min.) | Equilibrium Liquid-Suction Amount |
|---|---|---|---|---|---|---|---|
| | (g/g) | 2.07 kPa | 4.14kPa | 6.21 kPa | | | (g/g) |
| Ex.1 | 47 | 37 | 23 | 12 | 72 | 26 | 38 |
| Ex. 2 | 40 | 38 | 28 | 22 | 88 | 26 | 36 |
| Ex. 3 | 34 | 35 | 32 | 26 | 93 | 27 | 35 |
| Ex. 4 | 51 | 38 | 17 | 10 | 65 | 25 | 43 |
| Comp. Ex. 1 | 39 | 31 | 17 | 10 | 58 | 17 | 27 |
| Comp. Ex. 2 | 36 | 33 | 24 | 20 | 77 | 17 | 25 |
| Comp. Ex. 3 | 46 | 28 | 13 | 8 | 49 | 10 | 26 |

It can be seen from the results shown in Table 1 that all the water-absorbent resin particles obtained in each Example are excellent in the water absorption index under pressure, the liquid-suction rate, and the equilibrium liquid-suction amount from pulp even in the region of a relatively high water-retention capacity of physiological saline.

Example 5

Ten grams of the water-absorbent resin particles prepared in Example 1 and 10 g of pulverized wooden pulp were dry-blended, and the mixture was sprayed on tissue paper having sizes of 40 cm x 12 cm and a weight of 1 g. Thereafter, tissue paper having the same sizes and weight was layered over the top, and formed into a sheet. A 196 kPa load was applied to the entire sheet for 30 seconds to press the sheet, thereby giving an absorbent material. The resulting absorbent material was interposed between a polyethylene air-through non-woven fabric having a basis weight of 20 g/m$^2$ and a polyethylene sheet, thereby giving an absorbent article.

Examples 6 to 8 and Comparative Examples 4 to 6

The same procedures as in Example 5 were carried out except that in Example 5 the water-absorbent resin particles obtained in Examples 2 to 4 and Comparative Examples 1 to 3 were respectively used in place of the water-absorbent resin particles prepared in Example 1, to produce absorbent articles. The resulting absorbent articles were respectively named absorbent articles of Examples 6 to 8 and Comparative Examples 4 to 6 in that order.

Examples 9 and 10

The same procedures as in Examples 5 and 6 were carried out except that the amount of the water-absorbent resin particles was changed to 7 g, and that the amount of the wooden pulp was changed to 21 g in Examples 5 and 6, to produce absorbent articles. The resulting absorbent articles were respectively named absorbent articles of Examples 9 and 10 in that order.

Comparative Examples 7 and 8

The same procedures as in Examples 4 and 6 were carried out except that the amount of the water-absorbent resin particles was changed to 7 g, and that the amount of the wooden pulp was changed to 21 g in Comparative Examples 4 and 6, to produce absorbent articles. The resulting absorbent articles were respectively named absorbent articles of Comparative Examples 7 and 8 in that order.

The absorbent articles obtained in each Example and each Comparative Example were evaluated on the bases of the following methods. The results are shown in Table 2.

(5) Absorbent Article Test A

Composition of Artificial Urine: A 10 L container was charged with a proper amount of distilled water, and 60 g of sodium chloride, 1.8 g of calcium chloride dihydrate, and 3.6 g of magnesium chloride hexahydrate were added thereto to dissolve. Next, 0.02 g of polyoxyethylene nonyl phenyl ether was added to the solution, and distilled water was further added thereto to make up the mass of 6000 g of the entire mixture. Further, the mixture was colored with a small amount of Blue No. 1, to give an artificial urine.

A cylinder for liquid introduction having an inner diameter of 3 cm was placed at the central section of the absorbent article, and a stopwatch was clicked to start, and at the same time 50 mL of the artificial urine was introduced at one time,. The time from the start of the introduction to a timing where the liquid was completely absorbed in the absorbent article was determined, and referred to as a first permeation rate (seconds). Next, the same procedures were carried out at 30 minutes thereafter and 60 minutes thereafter to determine second and third permeation rates (seconds). After 120 minutes from the start of the introduction of the first liquid, 50 g of 10 cm x 10 cm filter papers were stacked near the liquid introductary point, and a 5.0 kg load was applied thereto for 5 minutes. The mass of the filter papers after applying the load was determined, and the mass of the filter papers before applying the load was subtracted therefrom, and the resulting value was defined as the amount of re-wet (g).

(6) Absorbent Article Test B

The same procedures as in Absorbent Article Test A were carried out except that the number of liquid introduction was changed to one time, that the amount of the artificial urine was changed to 150 mL, and that the load was started to be applied 5 minutes after the start of the first introduction.

[Table 2]

| Ex. No. and Comp. Ex No. | Absorbent Article Test A | | | | Absorbent Article Test B | |
|---|---|---|---|---|---|---|
| | Permeation Rate (seconds) | | | Amount of Re-wet (g) | Permeation Rate (seconds) | Amount of Re-wet (g) |
| | First | Second | Third | | | |
| Ex. 5 | 23 | 22 | 28 | 18.8 | 60 | 0.9 |
| Ex: 6 | 20 | 18 | 21 | 24.0 | 55 | 1.8 |
| Ex. 7 | 19 | 17 | 22 | 28.4 | 50 | 3.6 |
| Ex. 8 | 25 | 23 | 28 | 14.1 | 62 | 0.3 |
| Comp. Ex. 4 | 20 | 17 | 25 | 32.3 | 61 | 5.3 |
| Comp. Ex.5 | 18 | 16 | 21 | 35.1 | 59 | 8.2 |
| Comp. Ex. 6 | 23 | 23 | 35 | 29.8 | 75 | 2.1 |
| Ex. 9 | 10 | 10 | 13 | 32.4 | 32 | 2.3 |

(continued)

| Ex. No. and Comp. Ex No. | Absorbent Article Test A | | | | Absorbent Article Test B | |
|---|---|---|---|---|---|---|
| | Permeation Rate (seconds) | | | Amount of Re-wet (g) | Permeation Rate (seconds) | Amount of Re-wet (g) |
| | First | Second | Third | | | |
| Ex.10 | 10 | 9 | 12 | 34.1 | 32 | 7.4 |
| Comp. Ex.7 | 11 | 12 | 15 | 49.5 | 34 | 12.3 |
| Comp. Ex. 8 | 14 | 13 | 18 | 44.6 | 39 | 9.8 |

It can be seen from the results shown in Table 2 that all the absorbent articles obtained in each Example stably exhibited excellent properties even while the contents of the water-absorbent resin particles underwent various changes.

INDUSTRIAL APPLICABILITY

The absorbent material in which the water-absorbent resin particles of the present invention are used can be suitably used, for example, in absorbent articles such as disposable diapers and sanitary napkins.

**Claims**

1.  Water-absorbent resin particles, **characterized in that** the water-absorbent resin particles have a water-retention capacity of physiological saline (X) of from 30 to 70 g/g, that the water-retention capacity of physiological saline (X) and a water absorption index under pressure (Y) satisfy the relationship of the formula:

$$[\text{Water absorption index under pressure (Y)}] \geq 150 - 2[\text{Water-retention capacity of physiological saline (X)}],$$

and that the water-absorbent resin particles have a liquid-suction rate from pulp of 20 g/g/5 minutes or more.

2.  The water-absorbent resin particles according to claim 1, wherein the capacity of equilibrium liquid-suction amount from pulp is 30 g/g or more.

3.  An absorbent material comprising the water-absorbent resin particles as defined in claim 1 or claim 2, and a hydrophilic fiber.

4.  The absorbent material according to claim 3, wherein the water-absorbent resin particles are contained in an amount of from 5 to 80% by mass in the absorbent material.

5.  An absorbent article comprising the absorbent material as defined in claim 3 or claim 4 interposed between a liquid-permeable sheet and a liquid-impermeable sheet.

[Figure 1]

<div align="center">

**INTERNATIONAL SEARCH REPORT**

</div>

| | International application No. |
|---|---|
| | PCT/JP2005/020690 |

| A.    CLASSIFICATION OF SUBJECT MATTER |
|---|
| *C08F20/06*(2006.01), *A61F5/44*(2006.01), *A61F13/49*(2006.01), *A61F13/53*(2006.01), *A61L15/60*(2006.01), *B01J20/26*(2006.01), *C08F20/56*(2006.01) |
| According to International Patent Classification (IPC) or to both national classification and IPC |

| B.    FIELDS SEARCHED |
|---|
| Minimum documentation searched (classification system followed by classification symbols) |
| C08F20/06, A61F5/44, A61F13/49, A61F13/53, A61L15/60, B01F20/26, C08F20/56 |
| Documentation searched other than minimum documentation to the extent that such documents are included in the fields searched<br>    Jitsuyo Shinan Koho        1922-1996   Jitsuyo Shinan Toroku Koho   1996-2006<br>    Kokai Jitsuyo Shinan Koho   1971-2006   Toroku Jitsuyo Shinan Koho   1994-2006 |
| Electronic data base consulted during the international search (name of data base and, where practicable, search terms used)<br>    CA(STN) |

| C.    DOCUMENTS CONSIDERED TO BE RELEVANT | | |
|---|---|---|
| Category* | Citation of document, with indication, where appropriate, of the relevant passages | Relevant to claim No. |
| E,X | JP 2005-334616 A  (Sumitomo Seika Chemicals Co., Ltd.),<br>08 December, 2005 (08.12.05),<br>Claims; Par. Nos. [0034] to [0060], [0065] to [0092]; table 1<br>(Family: none) | 1-5 |
| A | JP 2004-290960 A  (Nippon Shokubai Co., Ltd.),<br>21 October, 2004 (21.10.04),<br>Claims<br>& WO 2004/069404 A1     & BR 200403937 A<br>& AU 2004210275 A1     & MX 2004009844 A1 | 1-5 |

| ☒  Further documents are listed in the continuation of Box C. | ☐  See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>    20 January, 2006 (20.01.06) | Date of mailing of the international search report<br>    31 January, 2006 (31.01.06) |
|---|---|
| Name and mailing address of the ISA/<br>    Japanese Patent Office | Authorized officer |
| Facsimile No. | Telephone No. |

Form PCT/ISA/210 (second sheet) (April 2005)

**INTERNATIONAL SEARCH REPORT**

| International application No. |
|---|
| PCT/JP2005/020690 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2004-121400 A  (San Daiya Polymer Kabushiki Kaisha), 22 April, 2004 (22.04.04), Claims; Par. No. [0101]] (Family: none) | 1-5 |
| A | JP 2003-192732 A  (San Daiya Polymer Kabushiki Kaisha), 09 July, 2003 (09.07.03), Claims (Family: none) | 1-5 |
| A | JP 2002-284805 A  (Mitsubishi Chemical Corp.), 03 October, 2002 (03.10.02), Claims; Par. Nos. [0013] to [0014], [0030] to [0041] (Family: none) | 1-5 |
| A | JP 2000-273107 A  (Mitsubishi Chemical Corp.), 03 October, 2000 (03.10.00), Claims; Par. Nos. [0013] to [0016]; table 1 (Family: none) | 1-5 |
| A | JP 10-244151 A  (Sanyo Chemical Industries, Ltd.), 14 September, 1998 (14.09.98), Claims; Par. Nos. [0022] to [0052] (Family: none) | 1-5 |
| A | JP 8-120018 A  (The Nippon Synthetic Chemical Industry Co., Ltd.), 14 May, 1996 (14.05.96), Claims; Par. Nos. [0026] to [0074] (Family: none) | 1-5 |

Form PCT/ISA/210 (continuation of second sheet) (April 2005)

**EP 1 813 628 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 11255837 A
- JP 6145253 A
- JP 8120013 A
- JP 6287233 A
- JP 2255804 A